# EUROPEAN PATENT APPLICATION

(11) **EP 4 763 070 A1**
(43) Date of publication of application: **24.06.2026**
(21) Application number: 24221440.1
(22) Date of filing: 19.12.2024
(51) Int. Cl.: A61B 5/053, A61B 5/0537, A61B 5/0538, A61B 5/00

(54) **IMPEDANCE SENSOR SYSTEM FOR LEAKS DETECTION AND GUIDED IMPLANTATION OF CARDIAC IMPLANTS**

(71) Applicant: Helmholtz-Zentrum hereon GmbH, 21502 Geesthacht (DE); Charité - Universitätsmedizin Berlin, 10117 Berlin (DE)
(72) Inventor: Reinthaler, Markus, 12203 Berlin (DE); Krasny, Marcin Jan, 12524 Berlin (DE); Polak-Krasna, Katarzyna, 12524 Berlin (DE)
(74) Representative: Uexküll & Stolberg

(57) **Abstract**

An implant device (2) has an outer surface (4) configured to be brought in contact with human or animal tissue. The outer surface (4) of the implant device (2) is equipped with one or more sensors (9), each of the one or more sensors (9) comprising an array (10) of at least two electrodes (11). Each of the electrodes (11) within the one or more sensors (9) is configured to emit and receive an electrical signal so as pairs of electrodes (11) measure a bioimpedance signal upon application of an electrical current or voltage. The implant device (2) further comprises a connecting hub (14) which is electrically connected to the at least two electrodes (11) and is electrically connectable to a circuit configured to convert the bioimpedance signal into an information on the contact between the implant device (2) and the human or animal tissue.

## Description

### FIELD OF INVENTION

The present disclosure relates generally to medical devices and more preferably to medical devices that are adapted for use in percutaneous medical procedures such as implantation into the left atrial appendage (LAA) of a heart.

### BACKGROUND OF THE INVENTION

Left atrial appendage occlusion (LAAO), also referred to as left atrial appendage closure (LAAC), is a treatment performed in high-risk patients for the prevention of thrombus formation in atrial fibrillation (AF) and for reducing the associated risk of stroke.

The left atrial appendage is a small organ attached to the left atrium of the heart. During normal heart function, as the left atrium constricts and forces blood into the left ventricle, the left atrial appendage constricts and forces blood into the left atrium. In patients suffering from atrial fibrillation, the left atrial appendage may not properly contract or empty, causing stagnant blood to pool within its interior, which can lead to the undesirable formation of thrombi within the left atrial appendage. Thrombi forming in the left atrial appendage may break loose from this area and enter the blood stream. Thrombi that migrate through the blood vessels may eventually plug a smaller vessel downstream and thereby contribute to stroke or an embolism in any other organ. Clinical studies have shown that the majority of blood clots in patients with atrial fibrillation originate in the left atrial appendage.

In the last 20 years, atrial fibrillation has become one of the most important public health problems and a significant cause of increasing health care costs in western countries. In these countries, subjects aged 60 to 70 years show an AF incidence of 3.7% to 4.2% which may increase up to 11% to 17% in those aged 80 years and older.

As stated, atrial fibrillation is associated with an increased risk of thromboembolic events, including stroke, which is the reason why patients are usually started on oral anticoagulation therapy. However, despite a broad range of anticoagulant options and improved uptake in anticoagulation over the past decade, there are limitations to this approach. First, there is a significant proportion of high-risk patients who have hematological disorders, frailty or previous major bleeding, whose high risk of bleeding precludes the use of anticoagulants. Second, there are patients who may continue to suffer thromboembolic events despite receiving appropriate guideline-directed anticoagulation therapy. Third, anticoagulation therapy can significantly decrease the quality of life in patients, who are prone to bruising and bleeding. Furthermore, compliance and adherence with drug therapy may be suboptimal; discontinuation rates in randomized controlled trials (RCTs) of direct oral anticoagulants (DOACs) are between 21% and 27%.

As an alternative treatment for patients suffering from atrial fibrillation, medical devices have been developed which are delivered to the site of implantation via a delivery catheter and then deployed to close the left atrial appendage.

The devices are usually delivered to the site of implantation percutaneously, whereby a delivery catheter comprising an outer sheath housing a compressed or collapsed implant is advanced from the femoral vein into the heart and into the left atrial appendage, and unfolded at the site of implantation. Current catheter diameters for the LAA device delivery are in the range of 11 to 16 Fr, which corresponds to 3.7 to 5.3 mm. The diameter range for the sealing area of the LAA is from 17 to 32 mm.

WO 2022/079235 A1 discloses a device to occlude the left atrial appendage of a heart of a subject comprising an implantable occlusion apparatus configured for radial expansion upon deployment to fluidically occlude the left atrial appendage, an elongated catheter member having a distal end attachable to the implantable occlusion apparatus for transluminal delivery of the implantable occlusion apparatus to the left atrial appendage. The device further comprises a tissue energizing module having a plurality of electrodes disposed around a circumference of the implantable occlusion apparatus in which each electrode is configured to contact a wall of the left atrial appendage at a tissue focal point upon deployment of the implantable occlusion apparatus, and an electrical controller including a pulsed field energy delivery generator operably attachable to an electrical power source and the plurality of electrodes and configured to energize the electrodes in a pulsed field ablation modality. The electrical controller is configured to independently energize each of the plurality of electrodes to apply a non-uniform pulsed field ablation treatment circumferentially around the wall of the left atrial appendage.

Co-pending application PCT/EP2024/071017 discloses an implant for occluding a left atrial appendage comprising a radially expansible and collapsible element configured to reversibly transition between a collapsed configuration and an expanded configuration. It further comprises a pair of core elements which extends in direction of a longitudinal axis, which have a tubular shape and which are rotatable relative to one another, a driving and locking element, a plurality of pairs of bendable spokes extending from the pair of core elements in an outward direction, and a plurality of shell elements attached to the outer end of the spokes and extending transversely in a curved manner so as to span a radial curved surface and forming a substantially circular outer circumference to be deployed against the wall of the left atrial appendage. The reversible transition between a collapsed configuration and an expanded configuration enables a controlled opening and repositioning of the LAAO during implantation in the LAA so as to achieve a proper uniform seal.

There is, however, a need to develop a device and a method to monitor the contact with the wall of human tissue of such medical implants enabling a contact determination at a local scale of the LAAO circumference. This is particularly useful for the above-described reversibly expandable and collapsible implant to enable a more reliable monitoring of the opening and repositioning of the LAAO during implantation in the LAA.

### SUMMARY OF THE INVENTION

The above object is achieved by an implant device having the features of claim 1. Preferred embodiments are set out in the dependent claims.

According to an aspect, the present disclosure relates to an implant device having an outer surface configured to be brought in contact with tissue, wherein the outer surface of the implant device is equipped with one or more sensors, each of the one or more sensors comprising an array of at least two electrodes, where each of the electrodes within the one or more sensors is configured to emit and receive an electrical signal so as pairs, triplets and or quadruplets of electrodes measure a bioimpedance signal upon application of an electrical current or voltage, and a connecting hub which is electrically connected to the at least two electrodes and is electrically connectable to a circuit configured to convert the bioimpedance signal into an information on the contact between the implant device and the tissue. Preferably, each sensor comprises an array of two, three, four or more electrodes.

Bioimpedance or biological impedance is defined as the ability of biological tissue to impede electric current. Impedance (Z), from an electrical point of view, is the obstruction to the flow of an alternating current and, hence, is dependent on the frequency of the applied current, defined in impedance magnitude (|Z|) and phase angle (φ). In biological systems impedance is caused by tissue structure and composition. Electrical impedance spectroscopy, a method used to electrically characterize biological matter, can be performed in a wide range of frequencies covering e.g. 1 mHz up to at least 1 MHz, preferably up to 100 kHz. Measuring signal parameters include an excitation voltage from microvolts (µY) to tens of volts, preferably 1 µV to 10 V and/or an excitation current from microamperes (µA) to tens of milliamperes (mA), preferably 1 µA to 10 mA. Bioimpedance or biological impedance relies on the fact that different types of tissues have varying electrical conductivities due to differences in composition and cell structure. By passing a low-level electrical current through the tissue and measuring the resulting voltage (or by applying voltage and by measuring the resulted current), bioimpedance devices can detect these differences. Based on the bioimpedance signal a differentiation can be made which allows to distinguish electrodes contacting blood pool from electrodes contacting other tissues. Additionally, a secondary information can be obtained which correlates bioimpedance with contact force between the implant device and the tissue as described above and additionally or alternatively to generate an information on the contact, preferably the contact force as a function of impedance. Thus, bioimpedance allows the positioning of the implant in the human or animal body such that proper contact between the outer surface of the implant device and the tissue in the human or animal body is established. Poor positioning of the implant with the risk of leaks between the implant device and the surrounding tissue as well as excessive contact pressure as a risk factor for perforation of the surrounding tissue can be avoided. Bioimpedance as described in connection with the present invention gives the surgeon feedback as how to position the implant device and whether the implant device needs to be repositioned. Also, the surgeon will be able to decide based on this feedback, how far the implant device is to be unfolded for optimal anchoring of the implant device to the tissue of the human or animal body.

In an embodiment, the outer surface comprises a circumferential portion extending along a longitudinal axis of the implant device and in a circumferential direction thereto. Preferably, the outer surface of the implant has a substantially semispherical or umbrella-like shape, wherein the sensors are arranged around the circumference of the outer surface, in particular on the circumferential portion. Preferably, the outer surface of the implant device is formed by a mesh, wherein each of the sensors is placed, preferably printed on the mesh. Preferably, the sensors are arranged in an equidistant manner around the circumference of the implant's outer surface, in particular on the circumferential portion. Multiple sensors may be distributed at equal distance on the outer surface of the device with minimal spacing between particular sensors necessary for high resolution bioimpedance measurement to deliver detailed feedback on device position and placement. Preferably, four, five, six, seven or eight sensors are arranged equidistantly around the implant's outer surface, in particular on the circumferential portion. Preferably, the electrodes of the sensors are made of an exposed conductive material, such as copper, stainless steel, gold, silver, platinum, nitinol, cobalt-chrome-molybdenum (Co-Cr-Mo) alloy, titanium, conductive polymers (e.g. PEDOT) or a combination thereof.

In an embodiment, the array of each of the one or more sensors comprises multiple electrodes which are arranged on the circumferential portion of the outer surface in a row, wherein the row of electrodes and the longitudinal axis of the implant devices when being projected onto the outer surface encloses an angle in a range of an absolute value of 0° to 90°, preferably in a range of 0° to 45°, more preferably of about 45°. An angled arrangement of the electrodes allows multiple measurements in the direction of the longitudinal axis of the implant device and at the same time in circumferential direction to the longitudinal axis of the implant device. Thus, the angled arrangement is an efficient arrangement of electrodes as it allows measurements in multiple direction with a comparatively low number of electrodes needed.

In an embodiment, the array of each of the one or more sensors comprises multiple electrodes which are arranged on the circumferential portion of the outer surface in a zigzag pattern, wherein the zigzag pattern includes a first row of electrodes and a second row of electrodes, wherein the two rows extend parallel to each other in a first direction, wherein the first row and the second row of electrodes are arranged offset to one another in the first direction such that each electrode of the first row is positioned between two adjacent electrodes of the second row when looking transverse to the first direction and wherein the first direction extends along the longitudinal axis of the implant device or transverse thereto in the circumferential direction. Such an arrangement increases the density of electrodes on the circumferential portion compared e.g. to the angled arrangement of the electrodes as described above. Thus, the electrodes being arranged in a zigzag pattern allow the generation of a bioimpedance signal and the information on the contact between the implant device and the human or animal tissue with a higher measurement resolution compared to the angled arrangement of the electrodes as described above.

In an embodiment, the array of each of the one or more sensors comprises multiple electrodes which are arranged on the circumferential portion of the outer surface in a matrix pattern, wherein the electrodes are arranged at a distance to each other in a direction parallel to the longitudinal axis and transverse thereto, and wherein the distances between the electrodes being arranged in a direction parallel to the longitudinal axis correspond to the distances of the electrodes being arranged in a direction transverse to the longitudinal axis. Such an arrangement increases the density of electrodes on the circumferential portion compared e.g. to the angled arrangement of the electrodes as described above. The electrodes being arranged in a matrix pattern allow the generation of a bioimpedance signal and the information on the contact between the implant device and the human or animal tissue with a higher measurement resolution compared to the angled arrangement of the electrodes as described above. However, the arrangement of the electrodes in a zigzag pattern as described above allows the highest measurement resolution compared to the matrix pattern and the angled arrangement of the electrodes.

In an embodiment, the electrodes are of circular, elliptical, square, rectangular, pentagonal, hexagonal, heptagonal or octagonal shape. Other electrode shapes are useful as well. Preferably, the diameter/size of each of the electrodes lies in a range from 1 µm to few centimeters, preferably 10 µm to 1 cm.

In an embodiment, the electrodes of each sensor are individually wired to the connecting hub. Preferably, each individual electrode of the at least two electrodes is connected to a source of electrical current or voltage. This allows a high degree of flexibility which pair of electrodes is applied with current or voltage to measure a bioimpedance signal. For example, if the surgeon when implanting the implant device suspects that there might be a weak contact (leak) between the implant device and the surrounding tissue at a certain position on the outer surface of the implant device, the surgeon might directly apply measurement procedure to focus on this specific area and rearrange measuring electrodes in the surrounding electrodes proximity to increase sensing resolution and accuracy to the position in question.

In an embodiment, the electrodes of each sensor are grouped into a first group of sensors and a second group of sensors, wherein the electrodes of the first group are connected in series to the connecting hub and, wherein the electrodes of the second group are individually connected to the connecting hub. Preferably, the electrodes of the first group which are connected in series may also be formed by an elongated single electrode. Preferably, the elongated single electrode has a length of around the circumference of the device, for example of up to 150 mm in case of an LAAO. This reduces the amount of connecting lines needed to electrically connect each of the electrodes to the connecting hub. Thus, connecting at least some of the electrodes in series to the connecting hub reduces costs and minimizes the risk of broken connecting lines. As regards the zigzag pattern, the first row of electrodes may form the first group of electrodes being connected in series whereas each of the electrodes of the second row may form the second group individually connected to the connecting hub. As regards the matrix pattern, e.g. electrodes arranged in a direction transverse to the longitudinal axis in an upper row form the first group of electrodes. The electrodes arranged below the first group of electrodes in a direction transverse to the longitudinal axis in a lower row form the second group of electrodes. The first group of electrodes and the second group of electrodes may be arranged in an alternating manner in a direction along the longitudinal axis.

In an embodiment, the outer surface of the implant device is at least partially formed by a flexible printed circuit board on which the electrodes of the at least one sensor are printed. Such a layout can be easily produced in a cost-efficient manner. Possible faults in the connecting lines, e.g. broken connecting lines, can be detected quickly and easily as all connecting lines are visible.

In an embodiment, the flexible printed circuit board comprises multiple layers, wherein the electrodes of the at least one sensor are printed on an outer layer of the multiple layers and connecting lines for electrically connecting each of the electrodes with the connecting hub are provided on one or more different layers located underneath the outer layer. This provides a space saving arrangement of the wiring, i.e. the connecting lines.

In an embodiment the flexible printed circuit board comprises multiple layers and the implant device comprises multiple sensors with the electrodes of the sensors being arranged on the outer layer of the multiple layers in a zigzag pattern as described above. The multiple layers further include multiple layers located underneath the outer layer, herein referred to as the "inner layers". The inner layers can be subdivided in a first row layer and multiple second row layers. The electrodes of the first row of electrodes of the zigzag pattern of the sensors are connected in series with the connecting hub by first connecting lines located on the first row layer of the multiple layers underneath the outer layer, wherein second connecting lines connecting the electrodes of the second row of electrodes of the zigzag pattern of the sensors with the connecting hub are located on second row layers of the multiple layers located underneath the outer layer, and each of the second row layers comprises the second connecting lines connecting the electrodes of one of the sensors with the connecting hub, and, wherein the electrodes of each one of the sensors are exclusively connected to the second connecting lines of one of the second row layers. In other words: Electrodes of the second row of a first sensor are connected to the second connecting lines on a first of the second row layers. Electrodes of the second row of a second sensor are connected to the second connecting lines on a second of the second row layers. As the first and second connecting lines are located on first and second of the second row layers, respectively, which are located underneath the outer layer, a space saving arrangement is achieved. For example, the second connecting lines connecting the electrodes of a first sensor may run on a first of the second row layers located above a second of the second row layers on which second connecting lines for connecting the electrodes of a second sensor are located.

In an embodiment, the flexible printed circuit board comprises multiple layers and the implant device comprises multiple sensors with the electrodes of each of the sensors being arranged in a zigzag pattern, wherein the electrodes of the first row of electrodes of the zigzag pattern of each of the sensors are connected in series and located on an outer layer of the multiple layers and, wherein the electrodes of the second row of electrodes of the zigzag pattern of each of the sensors together with corresponding connecting lines for electrically connecting each of the electrodes with the connecting hub are located on different layers located underneath the outer layer. Preferably, the electrodes of the first row of electrodes of the zigzag pattern of all sensors are connected in series by one single connecting line. Preferably, the electrodes of the first row of electrodes of the zigzag pattern of all sensors are provided by one common electrode, in particular by a single lengthy strip of electrodes. This reduces the number of connecting lines needed to connect the first row of electrodes with the connecting hub and thus the costs of the implant device. As the electrodes of the second row of electrodes are located on different layers located underneath the outer layer, a space saving arrangement of the second row of electrodes is provided. The electrodes may be arranged closer to each other than on a single layer printed circuit board thereby providing a higher measurement resolution compared to a single layer printed circuit board. Furthermore, the connecting paths may be arranged on separate layers allowing denser packing, thereby providing space saving in smaller devices compared to single layer solution.

In an embodiment, the flexible printed circuit board comprises multiple layers, wherein each of the electrodes of one sensor together with corresponding connecting lines for electrically connecting each of the electrodes with the connecting hub is arranged on a different layer, wherein the different layers are arranged offset to one another such that each of the electrodes located on a lower layer of the multiple layers the flexible printed circuit board is not covered by an adjacent upper layer of the multiple layers of the flexible printed circuit board. Preferably, an insulation layer is located between each of the layers comprising electrodes and connecting lines. This multilayer arrangement allows a dense packing of electrodes. Preferably, such a multilayer arrangement is manufactured by additive manufacturing such as screen printing or ink printing or aerosol printing.

In an embodiment, the flexible printed circuit board is stretchable and/or comprises recesses which allow the printed circuit board to deform in a curved manner. Deformability and/or stretchability of the printed circuit board increases the ability of the printed circuit board to adapt to the shape of the outer surface of the implant device, in particular the circumferential portion of the implant device. Preferably, the outer surface, in particular the circumferential portion comprises at least partly a curved shape, e.g. a cylindrical shape around the longitudinal axis of the implant device.

In an embodiment, the implant device is an occlusion device for occluding the left atrial appendage of the human heart, a heart valve replacement or repair device for opening vessels and/or covering aneurysms or a stent. The present invention may be used in connection with various implant devices which need a proper positioning as regards contact and/or corresponding contact force between the implant device and the surrounding tissue of the human body. Optionally, the device or system of the present invention may also be used in connection with other occluders and plugs which can be implanted in the human or animal body.

According to another aspect, the present disclosure relates to a system comprising the implant device as described above and a delivery catheter comprising a connecting interface to connect to the connecting hub of the implant device, wherein the delivery catheter further comprises wiring which electrically connects the one or more electrodes to the circuit configured to convert the bioimpedance signal into an information on the contact between the implant device and the human tissue. Preferably, the bioimpedance provides feedback to the surgeon as regards the position of the implant device and whether the implant needs to be repositioned. Also, the surgeon will be able to decide based on this feedback, how far the implant device is to be unfolded for optimal anchoring of the implant device to the tissue of the human body. Having reached the correct position, the connecting interface of the delivery catheter is disconnected from the connecting hub and the delivery catheter is removed from the human body. The implant device together with the sensors including the electrodes remains in the human body. The electrodes are preferably made of biocompatible and inert material (e.g. copper, stainless steel, gold, silver, platinum, nitinol, cobalt-chrome-molybdenum (Co-Cr-Mo) alloy, titanium, conductive polymers (e.g. PEDOT) or a combination thereof. Upon disconnection of the connecting interface from the connecting hub the electrodes are disconnected from the circuit configured to convert the bioimpedance signal into an information on the contact between the implant device and the human tissue. Thus, upon disconnection of the connecting interface from the connecting hub the electrodes are disconnected from any source of electrical current.

### DETAILED DESCRIPITON OF PREFERRED EMBODIMENTS

The present invention is described in detail in the following with reference to the figures, in which:
- Fig. 1: shows a perspective view of a system comprising an implant device with one or more sensors having an array of at least two electrodes;
- Fig. 2: shows a second embodiment of the array of electrodes arranged in a row;
- Fig. 3: shows a third embodiment of the array of electrodes arranged in a matrix pattern;
- Figs. 4a to 4c: show fourth to sixth embodiments of the array of electrodes with multiple alternative arrangements of first and second groups of electrodes;
- Fig. 5: shows a second embodiment of the implant device of Fig. 1 with the outer surface being at least partially formed by a flexible printed circuit board;
- Fig. 6: shows a second embodiment of the printed circuit board of Fig. 5; and
- Figs. 7a to 7b: show a third embodiment of the printed circuit board 24.

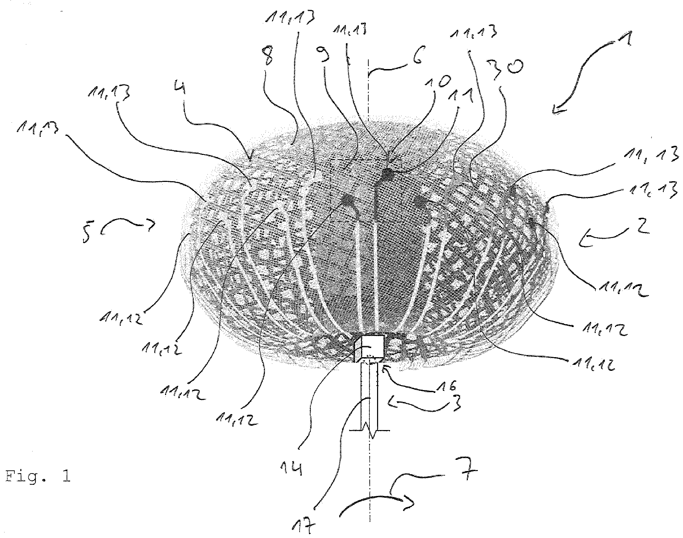

Figure 1 shows a perspective view of a system 1 comprising an implant device 2 and a delivery catheter 3. The implant device 2 is formed as an occlusion device for occluding the left atrial appendage of the human heart. The delivery catheter 3 is connectable to the implant device 2 to deliver it to the site of implantation and to subsequently deploy it to close the left atrial appendage. However, the present invention may also be used in combination with other implant devices such as a heart valve replacement or repair device for opening vessels and/or covering aneurysms or a stent.

The implant device 2 has an outer surface 4 configured to be brought in contact with human tissue. The outer surface 4 comprises a circumferential portion 5 extending along a longitudinal axis 6 of the implant device 2 and in a circumferential direction 7 thereto. The outer surface 4 of the implant device 2 has a substantially semispherical shape and is formed by a mesh 8. Preferably, the mesh 8 is stretched around a supporting structure 30 of the implant device 2.

The outer surface 4 of the implant device 2 is equipped with one or more sensors 9. Preferably, each of the sensors 9 is printed on the mesh 8. Each of the one or more sensors 9 comprises an array 10 of at least two electrodes 11 having a circular shape. However, the electrodes may also have an elliptical, square, rectangular, pentagonal, hexagonal, heptagonal or octagonal shape. Each of the electrodes 11 within the one or more sensors 9 is configured to emit and receive an electrical signal so as pairs of electrodes 11 generate a bioimpedance signal upon application of an electrical current.

As shown in Fig. 1 the array 10 of each of the multiple sensors 9 comprises two electrodes 11 which are arranged on the circumferential portion 7 of the outer surface 4 in a zigzag pattern. The zigzag pattern includes a first row 12 of electrodes 11 and a second row 13 of electrodes 11. The two rows 12, 13 extend parallel to each other in a first direction which is the circumferential direction 7. The first row 12 and the second row 13 of electrodes 11 are arranged offset to one another in the circumferential direction 7 such that each electrode 11 of the first row 12 is positioned between two adjacent electrodes 11 of the second row 13 when looking transverse to the circumferential direction 7.

The implant device 2 further comprises a connecting hub 14 which is electrically connected to the at least two electrodes 11. The connecting hub 14 is arranged along the longitudinal axis 6 and is preferably attached to the supporting structure 30 of the implant device 2. The electrodes 11 of each sensor 9 are individually wired to the connecting hub 14 via connecting lines 15. Preferably, the connecting lines 15 are also printed on the mesh 8. The delivery catheter 3 comprises a connecting interface 16 to connect to the connecting hub 14 of the implant device 2. The delivery catheter 3 further comprises wiring 17 which upon connection of the connecting interface 16 to the connecting hub 14 electrically connects the one or more electrodes 11 to a circuit (not shown) configured to measure a bioimpedance signal and convert it into an information on the contact between the implant device 2 and the human tissue. Each individual electrode 11 of the at least two electrodes 11 may be connected to a source of electrical current or voltage (not shown) via the delivery catheter 3 and the circuit.

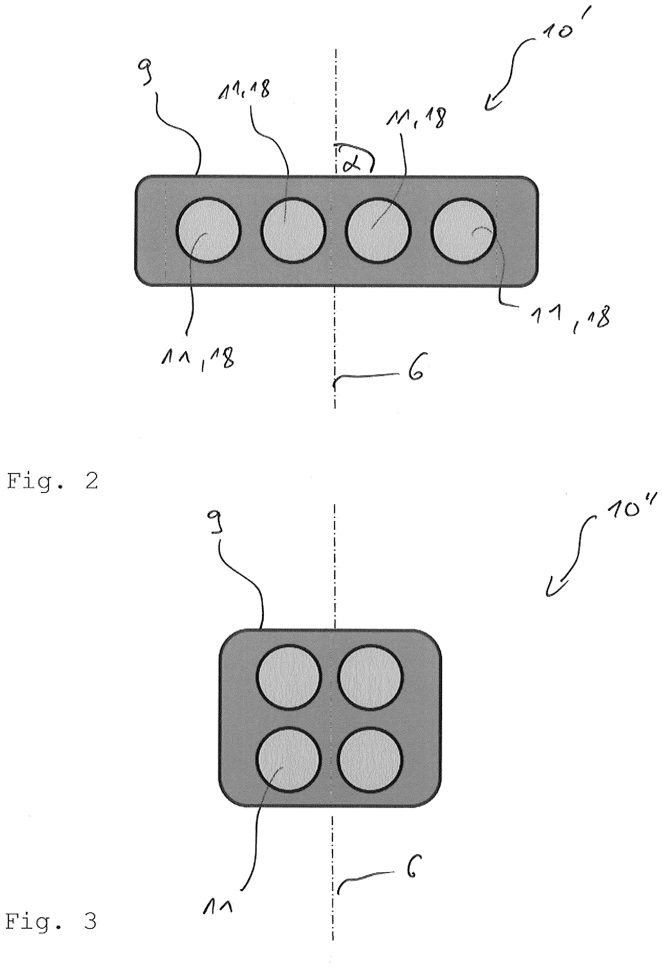

Fig. 2 shows a second embodiment of the array 10 of electrodes 11. The array 10' differs from the one as shown in Fig. 1 in that the array 10' of each of the one or more sensors 9 comprises multiple electrodes 11, in particular four electrodes 11, which are arranged on the circumferential portion 5 of the outer surface 4 (Fig. 1) in one single row 18. The single row 18 of electrodes 11 and the longitudinal axis 6 of the implant device 2 when being projected onto the outer surface 4 encloses an angle α in a range of 0° to 90°, preferably in a range of 0° to 45°. The angle α as shown in Fig. 2 is 90°. However, it is also preferred to arrange the single row 18 of electrodes 11 at an angle α of 45°. The array 10' may also comprise any other number of electrodes 11 such as 3, three, five, six and more.

Fig. 3 shows a third embodiment of the array 10 of electrodes 11. The array 10" differs from the one as shown in Fig. 1 in that the array 10‴ of each of the one or more sensors 9 comprises multiple electrodes 11, in particular four electrodes 11, which are arranged on the circumferential portion 5 of the outer surface 4 in a matrix pattern. The electrodes 11 are arranged at a distance to each other in a direction parallel to the longitudinal axis 6 and transverse thereto. The distances between the electrodes 11 being arranged in a direction parallel to the longitudinal axis 6 correspond to the distances of the electrodes being arranged in a direction transverse to the longitudinal axis 6.

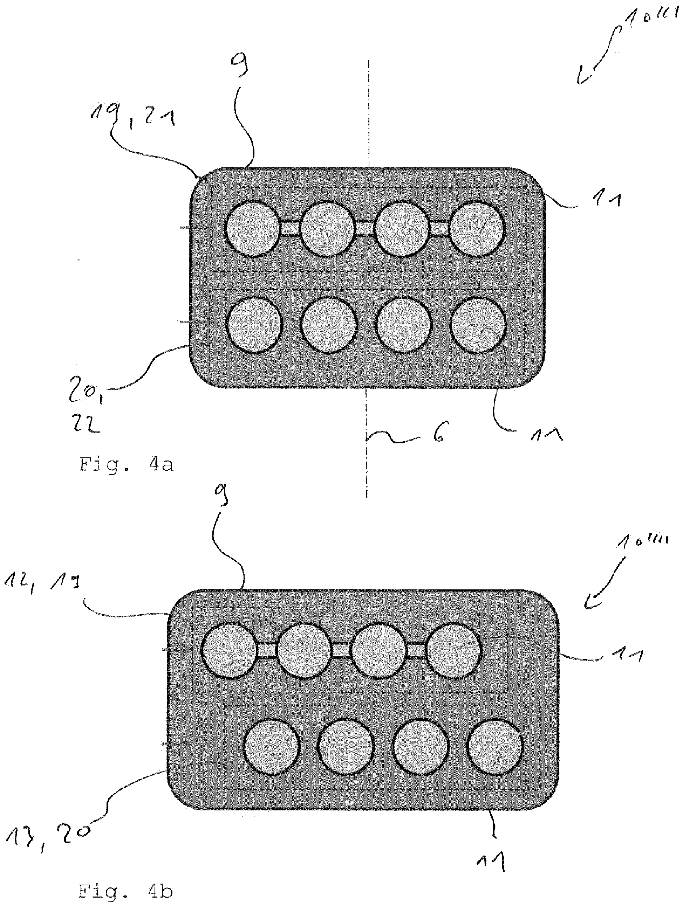

Figs. 4a to 4c show a fourth, a fifth and a sixth embodiment of the array 10, respectively. All of the fourth to sixth embodiments of the array 10 shown in Figs. 4a to 4c have in common that the electrodes 11 of each sensor 9 are grouped into a first group 19 of sensors 9 and a second group 20 of sensors 9. The electrodes 11 of the first group 19 are connected in series to the connecting hub 14 (Fig. 1) and the electrodes 11 of the second group 20 are individually connected to the connecting hub 14.

Fig. 4a relates to the matrix pattern of the electrodes 11 and shows the fourth embodiment of the array 10. The array 10‴ differs from the one as shown in Fig. 3 in that the electrodes 11 arranged in a direction transverse to the longitudinal axis 6 in an upper row 21 form the first group 19 of electrodes 11. The electrodes 11 arranged below the first group 19 of electrodes in a direction transverse to the longitudinal axis 6 in a lower row 22 form the second group 20 of electrodes 11. The first group 19 of electrodes and the second group 20 of electrodes may be arranged in an alternating manner in a direction along the longitudinal axis 6.

Fig. 4b relates to the zigzag pattern of the electrodes 11 and shows the fifth embodiment of array 10. The array 10'‴ differs from the one as shown in Fig. 1 in that the first row 12 of electrodes 11 forms the first group 19 of electrodes 11 being connected in series to the connecting hub 14, whereas each of the electrodes 11 of the second row 13 forms the second group 20 individually connected to the connecting hub 14.

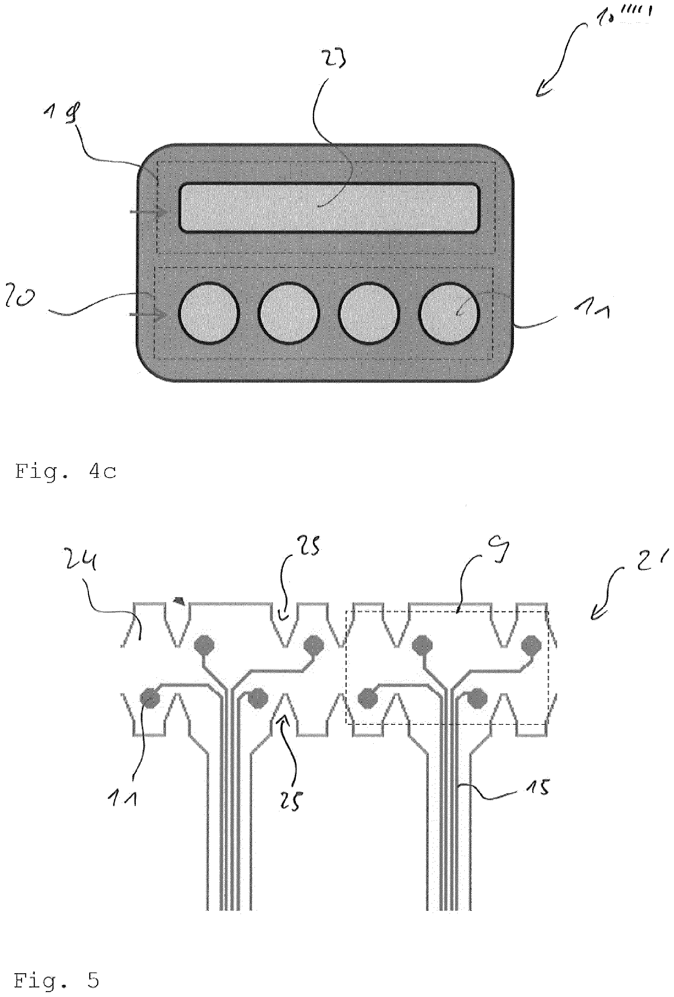

Fig. 4c shows a sixth embodiment of the array 10. The array 10‴ʺ differs from the one as shown in Fig. 4a in that the electrodes 11 of the first group 19 which are connected in series are formed by an elongated single electrode 23. The elongated single electrode 23 is connected to the connecting hub 14. It has to be understood that the use of an elongated single electrode 23 instead of multiple electrodes 11 connected in series is not limited to the matrix pattern of the electrodes 11 as shown in Fig. 4c but may also be applied to alternative embodiments of the array 10 of electrodes 11 as disclosed in connection with the present invention, such as the zigzag pattern as shown in Fig. 4b or the arrangement of the electrodes 11 in one single row 18 (see Fig. 2).

Fig. 5 partly shows a second embodiment of the implant device 2. The implant device 2' differs from the one as shown in Fig. 1 in that the outer surface 4 of the implant device 2' is at least partially formed by a flexible printed circuit board 24 on which the electrodes 11 of the at least one sensor 9 as well as the corresponding connecting lines 15 for connecting the electrodes 11 individually to the connecting hub 14 (Fig. 1) are printed. The flexible printed circuit board 24 is stretchable and/or comprises recesses 25 which allow the printed circuit board 24 to deform in a curved manner.

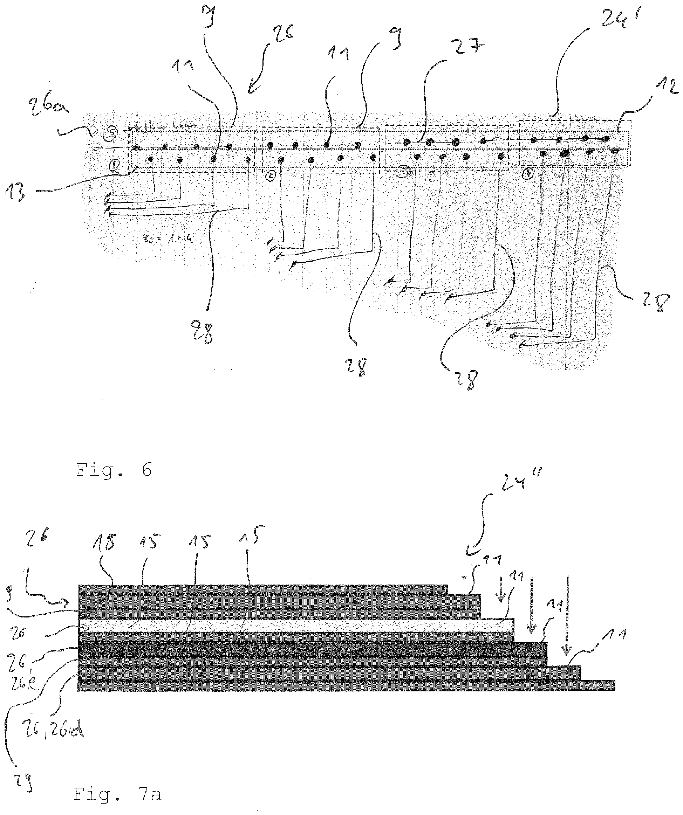

Fig. 6 shows a second embodiment of the printed circuit board 24. The printed circuit boards 24' differs from the one as shown in Fig. 5 in that the flexible printed circuit board 24' comprises multiple layers 26 and the implant device 2 comprises multiple sensors 9 with the electrodes 11 of the sensors 9 being arranged on an outer layer 26a of the multiple layers 26 in a zigzag pattern. The electrodes 11 of the first row 12 of electrodes 11 of the zigzag pattern of the sensors 9 are connected in series with the connecting hub 14 by a first connecting line 27 located on a first row layer 26b of the multiple layers 26 underneath the outer layer 26a. Preferably, the first row layer 26b forms the bottom layer of the printed circuit board 24'. Second connecting lines 28 connecting the electrodes 11 of the second row 13 of electrodes 11 of the zigzag pattern of the sensors 9 with the connecting hub 13 are located on second row layers 26c of the multiple layers 26 located underneath the outer layer 26a. Preferably, the second row layers 26c are arranged underneath the outer layer 26a and above the bottom layer formed by the first row layer 26b. Each of the second row layers 26c comprises the second connecting lines 28 connecting the electrodes 11 of one of the sensors 9 with the connecting hub 14, wherein the electrodes 11 of each one of the sensors 9 are exclusively connected to the second connecting lines 28 of one of the second row layers 26c.

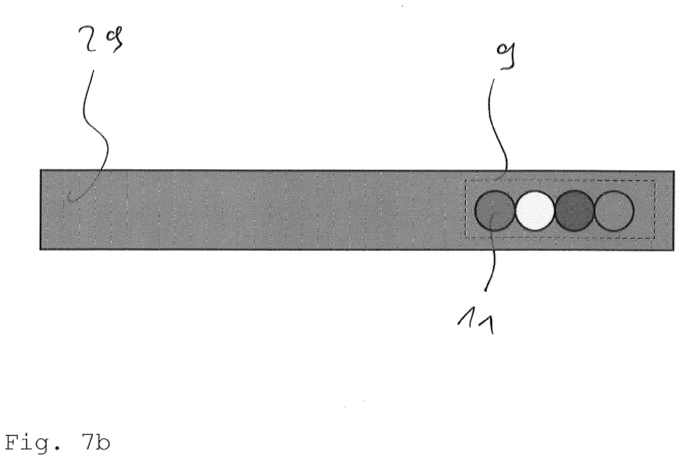

Fig. 7a and 7b show a third embodiment of the printed circuit board 24. The printed circuit board 24" differs from the one shown in Fig. 5 in that the flexible printed circuit board comprises multiple layers 26, wherein each of the electrodes 11 of one sensor 9 together with corresponding connecting lines 15 for electrically connecting each of the electrodes 11 with the connecting hub 14 is arranged on a different layer of the multiple layers 26. The different layers of the multiple layers 26 are arranged offset to one another such that each of the electrodes 11 located on a first lower layer 26d of the multiple layers 26 is not covered by an adjacent second upper layer 26e of the multiple layers 26. An insulation layer 29 is located between each of the layers 26 comprising electrodes 11 and connecting lines 15. Fig. 7a shows a side view of the printed circuit board 24". Fig. 7b shows a top view of the printed circuit board 24".

### Reference numerals

1. System
2, 2` Implant device
3. Delivery catheter
4. Outer surface
5. Circumferential portion
6. Longitudinal axis (implant device)
7. Circumferential direction (longitudinal axis)
8. Mesh
9. Sensor
10, 10', 10"', 10"`, 10‴ʺ Array
11. Electrode
12. First row (electrode)
13. Second row (electrodes)
14. Connecting hub (implant device)
15. Connecting lines
16. Connecting interface (delivery catheter)
17. Wiring (delivery catheter)
18. Single row (electrodes)
α Angle
19. First group
20. Second group
21. Upper row (matrix pattern)
22. Lower row (matrix pattern)
23. Elongated single electrode
24, 24', 24" printed circuit board
25. Recess (printed circuit board)
26. Layer (printed circuit board)
26a. Outer layer (printed circuit board)
26b. First row layer (printed circuit board)
26c. Second row layers (printed circuit board
26d. First lower layer (printed circuit board)
26e. Second upper layer (printed circuit board)
27. First connecting line
28. Second connecting line
29. Insulation layer
30. Supporting structure

## Claims

1. An implant device having
an outer surface (4) configured to be brought in contact with human or animal tissue,
wherein the outer surface (4) of the implant device (2, 2') is equipped with one or more sensors (9), each of the one or more sensors (9) comprising an array (10, 10', 10", 10"', 10"", 10‴ʺ) of at least two electrodes (11), where each of the electrodes (11) within the one or more sensors (9) is configured to emit and receive an electrical signal so as pairs of electrodes (11) measure a bioimpedance signal upon application of an electrical current or voltage, and
a connecting hub (14) which is electrically connected to the at least two electrodes (11) and is electrically connectable to a circuit configured to convert the bioimpedance signal into an information on the contact between the implant device (2, 2') and the human or animal tissue.

2. The implant device of claim 1, wherein the outer surface (4) comprises a circumferential portion (5) extending along a longitudinal axis (6) of the implant device (2, 2') and in a circumferential direction thereto.

3. The implant device of claim 2, wherein the array (10') of each of the one or more sensors (9) comprises multiple electrodes (11) which are arranged on the circumferential portion (5) of the outer surface (4) in a row (18), wherein the row (18) of electrodes (11) and the longitudinal axis (6) of the implant device (2, 2') when being projected onto the outer surface (4) encloses an angle (α) in a range of an absolute value of 0° to 90°, preferably in a range of 0° to 45°, more preferably of about 45°.

4. The implant device of claim 2, wherein the array (10‴) of each of the one or more sensors (9) comprises multiple electrodes (11) which are arranged on the circumferential portion (5) of the outer surface (4) in a zigzag pattern, wherein the zigzag pattern includes a first row (12) of electrodes (11) and a second row (13) of electrodes (11), wherein the two rows (12, 13) extend parallel to each other in a first direction, wherein the first row (12) and the second row (13) of electrodes (11) are arranged offset to one another in the first direction such that each electrode (11) of the first row (12) is positioned between two adjacent electrodes (11) of the second row (13) when looking transverse to the first direction and wherein the first direction extends along the longitudinal axis (6) of the implant device (2, 2') or transverse thereto in the circumferential direction (7).

5. The implant device of claim 2, wherein the array (10") of each of the one or more sensors (9) comprises multiple electrodes (11) which are arranged on the circumferential portion (5) of the outer surface (4) in a matrix pattern, wherein the electrodes (11) are arranged at a distance to each other in a direction parallel to the longitudinal axis (6) and transverse thereto, and wherein the distances between the electrodes (11) being arranged in a direction parallel to the longitudinal axis (6) correspond to the distances of the electrodes (11) being arranged in a direction transverse to the longitudinal axis (6).

6. The implant device of any one of the preceding claims, wherein the electrodes (11) are of circular, elliptical, square, rectangular, pentagonal, hexagonal, heptagonal or octagonal shape.

7. The implant device of any one of the preceding claims, wherein the electrodes (11) of each sensor (9) are individually wired to the connecting hub (14).

8. The implant device of any one of claims 1 to 6, wherein the electrodes (11) of each sensor (9) are grouped into a first group (19) of sensors (9) and a second group (20) of sensors (9), wherein the electrodes (11) of the first group (19) are connected in series to the connecting hub (14) and, wherein the electrodes (11) of the second group (20) are individually connected to the connecting hub (14).

9. The implant device of any one of the preceding claims, wherein the outer surface (4) of the implant device (2') is at least partially formed by a flexible printed circuit board (24, 24') on which the electrodes (11) of the at least one sensor (9) are printed.

10. The implant device of claim 9, wherein the flexible printed circuit board (24') comprises multiple layers (26), wherein the electrodes (11) of the at least one sensor (9) are printed on an outer layer (26a) of the multiple layers (26) and connecting lines (15) for electrically connecting each of the electrodes (11) with the connecting hub (14) are provided on one or more different layers (26) located underneath the outer layer (26a).

11. The implant device of claims 4 and 9, wherein the flexible printed circuit board (24') comprises multiple layers (26) and the implant device (2, 2') comprises multiple sensors (9) with the electrodes (11) of the sensors (9) being arranged on an outer layer (26) of the multiple layers (26) in a zigzag pattern, wherein the electrodes (11) of the first row (12) of electrodes (11) of the zigzag pattern of the sensors (9) are connected in series with the connecting hub (14) by a first connecting line (27) located on a first row layer (26b) of the multiple layers (26) underneath the outer layer (26a), wherein second connecting lines (28) connecting the electrodes (11) of the second row (13) of electrodes (11) of the zigzag pattern of the sensors (9) with the connecting hub (14) are located on second row layers (26c) of the multiple layers (26) located underneath the outer layer (26), wherein each of the second row layers (26c) comprises the second connecting lines (28) connecting the electrodes (11) of one of the sensors (9) with the connecting hub (14) and, wherein the electrodes (11) of each one of the sensor (9) are exclusively connected to the second connecting lines (28) of one of the second row layers (26c).

12. The implant device of claim 9, wherein the flexible printed circuit board (24") comprises multiple layers (26), wherein each of the electrodes (11) of one sensor (9) together with corresponding connecting lines (15) for electrically connecting each of the electrodes (11) with the connecting hub (14) is arranged on a different layer (26) of the multiple layers (26), wherein the different layers (26) are arranged offset to one another such that each of the electrodes (11) located on a first lower layer (26d) of the multiple layers (26) is not covered by an adjacent second upper layer (26e) of the multiple layers (26).

13. The implant device of claim 9, wherein the flexible printed circuit (24, 24', 24") board is stretchable and/or comprises recesses (25) which allow the printed circuit (24, 24', 24") board to deform in a curved manner.

14. The implant device of any one of the preceding claims, wherein the implant device (2, 2') is an occlusion device for occluding the left atrial appendage of the human heart, a heart valve replacement or repair device for opening vessels and/or covering aneurysms or a stent.

15. A system comprising:
the implant device (2, 2') according to any of claims 1 to 14 and
a delivery catheter (3) comprising a connecting interface (16) to connect to the connecting hub (14) of the implant device (2, 2'),
wherein the delivery catheter (3) further comprises wiring (17) which electrically connects the one or more electrodes (11) to the circuit configured to convert the bioimpedance signal into an information on the contact between the implant device (2, 2') and the human tissue.
